# EUROPEAN PATENT APPLICATION

(11) **EP 3 459 481 A1**
(43) Date of publication of application: **27.03.2019**
(21) Application number: 18195609.5
(22) Date of filing: 20.09.2018
(51) Int. Cl.: A61B 18/14, A61B 18/00, A61B 17/285, A61B 18/12

(54) **ELECTROSURGICAL INSTRUMENT FOR CONTINUOUS TISSUE SECTIONING**

(30) Priority: 21.09.2017 US 201762561250 P; 20.07.2018 US 201816040784
(71) Applicant: Covidien LP, Mansfield, MA 02048 (US)
(72) Inventor: BEGG, Nikolai, D., Wayland, MA Massachusetts 01778 (US)
(74) Representative: Maschio, Antonio

(57) **Abstract**

A surgical system includes an electrosurgical instrument having a housing, a shaft extending distally from the housing, an electrosurgical loop adapted to connect to a source of energy and operably supported at a distal end portion of the shaft, and a bumper extending from the distal end portion of the shaft at an angle relative to the electrosurgical loop. A grasping device includes a handle assembly, a shaft assembly, and an end effector assembly configured for grasping tissue. The grasping device is configured to pull tissue through the electrosurgical loop such that the tissue is pulled into contact with the electrosurgical loop.

## Description

### BACKGROUND

### Technical Field

The present disclosure relates to electrosurgical instruments, and more particularly, to electrosurgical loop instruments for resecting tissue.

### Background of Related Art

Several minimally-invasive surgical procedures (e.g., laparoscopic) require the removal of body tissue or organs through a limited access opening. As such, the tissue needs to be broken down within the body cavity to facilitate removal through the limited access opening. In some instances, it is advantageous to break down the tissue into one or more large tissue segments, rather than small tissue segments, to enable removal in fewer extraction steps. Apart from time savings, the removal of large tissue segments, rather than small tissue segments, reduces the chance of cross-contamination with malignant or cancerous tissue.

### SUMMARY

Accordingly, a need exists for an electrosurgical instrument that can continuously section large tissue segments.

According to an aspect of the present disclosure, an electrosurgical instrument is provided including a housing, a shaft extending distally from the housing, an electrosurgical loop configured to connect to a source of energy and operably supported at a distal end portion of the shaft, and a bumper extending from the distal end portion of the shaft at an angle relative to the electrosurgical loop.

In embodiments, the electrosurgical loop defines an active electrode and the bumper defines a return electrode in a bipolar configuration.

In some embodiments, the bumper is formed from a metal selected from the group consisting of copper, copper alloy, stainless steel, tungsten, platinum, niobium, titanium, steel, and aluminum.

In certain embodiments, the shaft defines a first axis and the bumper defines a second axis that is oriented at an angle from about 75 degrees to about 120 degrees relative to the first axis.

In embodiments, the bumper is pivotable relative to the shaft between a rest position and a pivoted position.

In some embodiments, the electrosurgical instrument includes biasing element having a first end and a second end. The first end of the biasing element is attached to the bumper and the second end of the biasing element is attached to the shaft. The bumper is configured to move to the pivoted position when in contact with a surface and configured to automatically return to the rest position when not in contact with a surface.

In certain embodiments, the electrosurgical loop is movable between a contracted position, wherein the electrosurgical loop defines a first dimension, and an expanded position, wherein the electrosurgical loop defines a second, larger dimension.

In embodiments, the electrosurgical instrument includes an actuator operably coupled to the housing and a drive assembly extending through the shaft and operably coupling the actuator with the electrosurgical loop. Actuation of the actuator moves the electrosurgical loop between the contracted and expanded positions.

In some embodiments, the housing includes an activation button on a surface thereof. The activation button is configured to selectively energize the electrosurgical loop with the source of electrical energy.

In certain embodiments, the bumper defines a blunt free end.

In embodiments, a distal tissue contacting surface of the bumper defines one of a flat, smooth, rough, and textured surface.

According to another aspect of the present disclosure, a method of performing a surgical procedure is provided, including inserting an electrosurgical instrument including an electrosurgical loop and a bumper disposed at an angle relative to the electrosurgical loop into a body cavity. The method includes activating an activation button to energize the electrosurgical loop, drawing tissue from a tissue specimen through the electrosurgical loop to resect a strip of tissue from the tissue specimen, and sliding the bumper along a surface of the tissue specimen while drawing the tissue to facilitate resection of the strip of tissue.

In embodiments, drawing tissue includes grasping tissue with a grasping device and pulling tissue with the grasping device through the electrosurgical loop.

In some embodiments, the method includes actuating an actuator of the electrosurgical instrument to expand or contract the electrosurgical loop.

According to yet another aspect of the present disclosure, a surgical system is provided including an electrosurgical instrument having a housing, a shaft extending distally from the housing, an electrosurgical loop adapted to connect to a source of energy and operably supported at a distal end portion of the shaft, and a bumper extending from the distal end portion of the shaft at an angle relative to the electrosurgical loop. A grasping device is provided and includes a handle assembly, a shaft assembly, and an end effector assembly configured for grasping tissue. The grasping device is configured to pull tissue through the electrosurgical loop such that the tissue is pulled into contact with the electrosurgical loop.

In embodiments, the electrosurgical loop defines an active electrode and the bumper defines a return electrode in a bipolar configuration.

In some embodiments, the bumper is formed from a metal selected from the group consisting of copper, copper alloy, stainless steel, tungsten, platinum, niobium, titanium, steel, and aluminum.

In certain embodiments, the shaft defines a first axis and the bumper defines a second axis that is oriented at an angle from about 75 degrees to about 120 degrees relative to the first axis.

In embodiments, the bumper is pivotable relative to the shaft between a rest position and a pivoted position.

In some embodiments, the electrosurgical loop is movable between a contracted position, wherein the electrosurgical loop defines a first dimension, and an expanded position, wherein the electrosurgical loop defines a second, larger dimension.

### BRIEF DESCRIPTION OF THE DRAWINGS

Objects and features of the present disclosure will become apparent to those of ordinary skill in the art when descriptions thereof are read with reference to the accompanying drawings, of which:
FIG. 1 is a perspective view of an electrosurgical instrument in accordance with the present disclosure;
FIG. 2A is a side view of a distal end portion of the electrosurgical instrument of FIG. 1;
FIG. 2B is a top view of the distal end portion of the electrosurgical instrument of FIG. 1;
FIG. 3A is a side view of the distal end portion of the electrosurgical instrument of FIG. 1 in an expanded position;
FIG. 3B is a top view of the distal end portion of the electrosurgical instrument of FIG. 1 in the expanded position;
FIG. 4A is a side view of the distal end portion of the electrosurgical instrument of FIG. 1 in a contracted position;
FIG. 4B is a top view of the distal end portion of the electrosurgical instrument of FIG. 1 in the contracted position;
FIG. 5A is a schematic diagram of the distal end portion of the electrosurgical instrument of FIG. 1 and a grasping instrument in contact with tissue; and
FIG. 5B is a schematic diagram of the grasping instrument of FIG. 5A pulling a strip of tissue through the distal end portion of the electrosurgical instrument of FIG. 1.

### DETAILED DESCRIPTION

Embodiments of the present disclosure are described in detail with reference to the drawings in which like reference numerals designate identical or corresponding elements in each of the several views. As used herein, the term "distal" refers to that portion of structure farther from the user, while the term "proximal" refers to that portion of structure closer to the user. As used herein, the term "clinician" refers to a doctor, nurse, or other care provider and may include support personnel. In the following description, well-known functions or constructions are not described in detail to avoid obscuring the present disclosure in unnecessary detail.

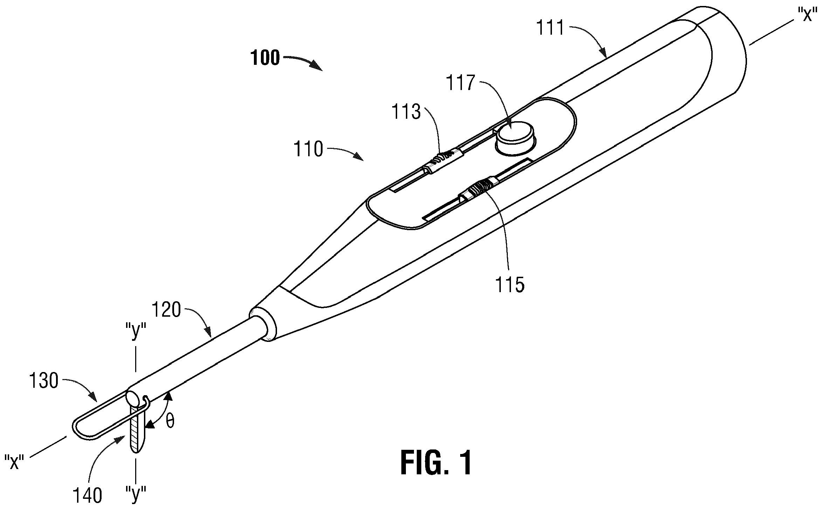

Referring initially to FIG. 1, an electrosurgical instrument in accordance with the present disclosure is shown and designated as 100. Electrosurgical instrument 100 is configured for insertion in a body cavity (e.g., abdominopelvic cavity) through a natural orifice, incision, and/or port (e.g., vaginal, abdominal, etc.) to resect tissue (e.g., uterine fibroids, cancerous cells, etc.) therein, although electrosurgical instrument 100 may also be used in open surgical procedures and/or for other suitable purposes. Electrosurgical instrument 100 defines a longitudinal axis "X-X" and generally includes a housing 110, a shaft 120 extending distally from handle assembly 110, an electrosurgical loop 130 supported at a distal end portion of shaft 120, and a bumper 140 supported at the distal end portion of shaft 120 and operably positioned relative to electrosurgical loop 130, as detailed below.

Housing 110 of electrosurgical instrument 100 defines a handle portion 111 configured to enable gripping and manipulation of electrosurgical instrument 100. First and second actuators 113, 115 are operably coupled to housing 110, as is an activation button 117. First and second actuators 113, 115 are disposed on opposing sides of housing 110 and operably connected to electrosurgical loop 130. Either or both of first and second actuators 113, 115 (depending upon clinician preference, handedness, etc.) is selectively actuatable to move electrosurgical loop 130, as described below. Activation button 117 is selectively activatable to energize electrosurgical loop 130, as also described below.

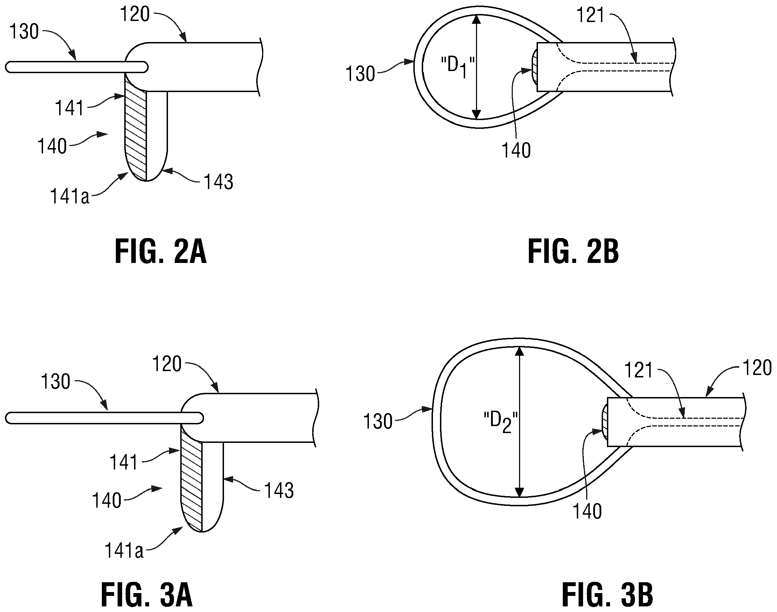

Shaft 120 extends distally from housing 110 and is configured for insertion through an access opening into a body cavity, e.g., an abdominopelvic cavity. Shaft 120 includes a drive assembly 121 (FIG. 2B) extending therethrough. Drive assembly 121 is connected to first and second actuators 113, 115 at a proximal end portion thereof and to electrosurgical loop 130 at a distal end portion thereof. As such, actuation of either or both of first and second actuators 113, 115 moves drive assembly 121 through shaft 120 to move electrosurgical loop 130, as described below. Although actuators 113, 115 are illustrated as slide-actuators, any other suitable actuator(s) may alternatively be provided. Drive assembly 121 may include cables, linkages, rods, or other suitable component(s) operably coupled between first and second actuators 113, 115 and electrosurgical loop 130 to enable movement of electrosurgical loop 130.

With additional reference to FIGS. 2A-4B, electrosurgical loop 130 is operatively supported at a distal end portion of shaft 120. Electrosurgical loop 130 extends distally from the distal end portion of shaft 120 in a longitudinal direction, although other orientations of electrosurgical loop 130 may also be provided. Electrosurgical loop 130 is electrically coupled to activation button 117 and configured to connect to a source of electrosurgical energy (not shown) to enable selective energization of electrosurgical loop 130 for resecting tissue therewith, e.g., upon activation of activation button 117. Electrosurgical loop 130 is configured for encircling tissue (e.g., snaring, grasping, etc.) to resect tissue at a target site and may also be used to move or displace tissue.

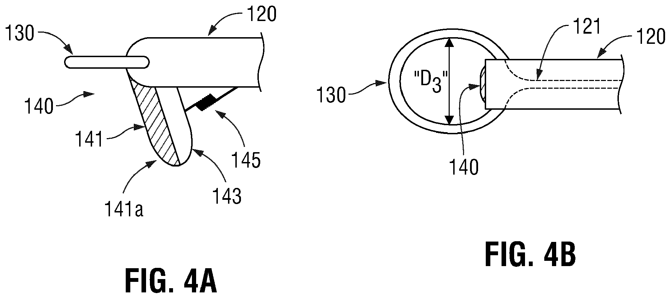

Movement of either of both of first and second actuators 113, 115 alters (e.g., expands or contracts) a dimension of electrosurgical loop 130. More specifically, movement of either of both of first and second actuators 113, 115 causes electrosurgical loop 130 to move from a default position (FIGS. 2A and 2B), wherein electrosurgical loop 130 defines a first dimension "Di" (FIG. 2B), to an expanded position (FIGS. 3A and 3B), wherein electrosurgical loop 130 defines a second dimension "D₂" (FIG. 3B) that is greater than the first dimension "D₁," or a contracted position (FIGS. 4A and 4B), wherein electrosurgical loop 130 defines a third dimension "D₃" (FIG. 4B) that is less than the first and second dimensions "D₁" and "D₂," depending upon a direction of movement of first and/or second actuator 113, 115. Adjusting the dimension of electrosurgical loop 130 facilitates resecting, e.g., larger or smaller strips of tissue, cinching tissue to separate a strip of tissue from a tissue specimen, ensnaring tissue, etc. The adjusted dimension of electrosurgical loop 130 may be a length, width, diameter, or other suitable dimension.

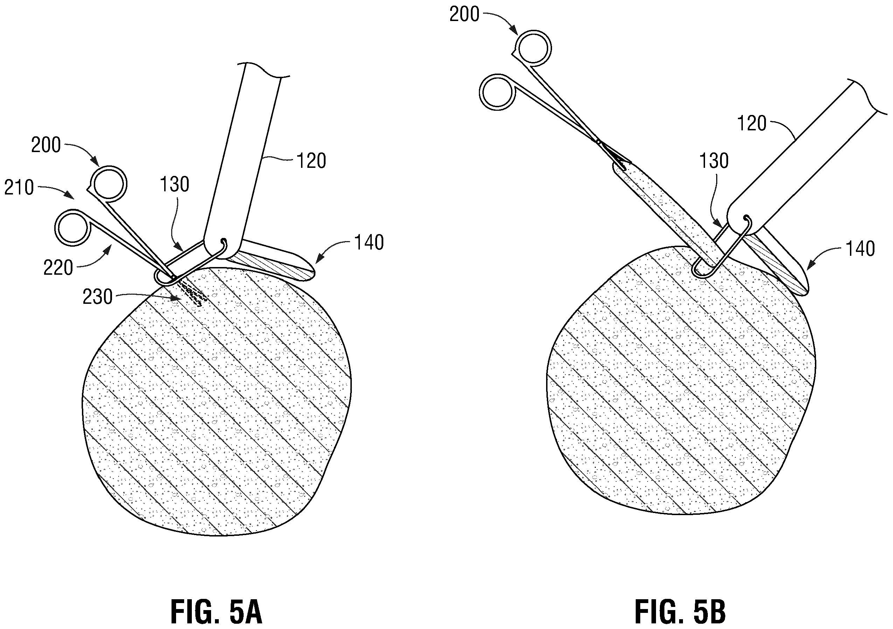

It should be appreciated that electrosurgical loop 130 is configured to resect tissue in any of the positions mentioned. Additionally or alternatively, a clinician may manipulate handle 111 of housing 110 to provide the clinician with additional control for maneuvering electrosurgical loop 130 into position or, when energized, through tissue to resect tissue, and/or electrosurgical loop 130 may be configured to move (e.g., articulate, rotate, pivot, etc.) relative to axis "X-X" defined by electrosurgical instrument 100 for similar purposes. Electrosurgical loop 130 may further be configured to resiliently flex from its at-rest position (see FIGS. 5A and 5B) to enable electrosurgical loop 130 to conform to and maintain contact with tissue (under bias), as detailed below.

Electrosurgical loop 130 may be configured to receive monopolar energy and serve as an active electrode for use with a remote return pad (not shown) to conduct energy through tissue to resect tissue. Alternatively, electrosurgical loop 130 may define one electrode in a bipolar configuration with an electrically-isolated component of electrosurgical instrument 100, such as bumper 140, serving as the return electrode to enable conduction of energy therebetween and through tissue to resect tissue.

Electrosurgical loop 130 may be formed as a wire having any suitable cross-sectional configuration, e.g., circular, semi-circular, triangular, rectangular, oval, polygonal, etc. Electrosurgical loop 130 may be formed from or include any material having suitable electrical conductivity. For example, electrosurgical loop 130 may be formed from metal, such as, e.g., copper, copper alloy, stainless steel, tungsten, platinum, niobium, molybdenum, etc.

Bumper 140 generally includes a tissue contacting surface 141, a blunt free end 141a, and a proximal surface 143. Bumper 140 defines an axis "Y-Y" that is angled relative to shaft 120 and electrosurgical loop 130. It is contemplated that bumper 140 be oriented at any suitable angle θ (e.g., from about 75 degrees to about 120 degrees) relative to shaft 120 and/or electrosurgical loop 130. Tissue contacting surface 141 may include a smooth or flat surface to facilitate movement of bumper 140 along a tissue surface, or alternatively, a rough surface, textured surface, toothed-surface, etc., to provide traction against movement along a tissue surface. Additionally or alternatively, bumper 140 may include a liner or pad disposed thereon, which may itself include a smooth surface, textured surface, etc.

Bumper 140 may be fixedly attached to shaft 120 or may be pivotably attached to shaft 120 (e.g., via a living a hinge, pivot pin, flexible connection, etc.) such that bumper 140 may pivot (FIG. 4A) relative to shaft 120 and electrosurgical loop 130. In embodiments, a biasing element 145 (FIG. 4A) may be attached at a first end thereof to bumper 140 and at a second end thereof to shaft 120. Biasing element 145 biases bumper 140 relative to shaft 120 towards an at-rest orientation. Alternatively, such bias may be provided via a living hinge or other suitable connection that couples bumper 140 to shaft 120. In either configuration, bumper 140 is configured to move from an at-rest position to a pivoted position when in contact with a surface (e.g., tissue) to conform to and maintain contact with tissue (under bias). Bumper 140 may return to the at-rest position when not in contact with a surface, under the bias of biasing element 145 or the connection between bumper 140 and shaft 120.

Bumper 140 may have any suitable cross-sectional shape, such as rectangular, semi-circular, spherical, etc., or combinations thereof. Bumper 140 may be formed from any suitable material, such as copper, copper alloy, stainless steel, tungsten, platinum, niobium, titanium, steel, aluminum, etc. As discussed above, in embodiments, electrosurgical instrument 100 defines a bipolar configuration, wherein bumper 140 functions as a return electrode. In such embodiments, tissue contacting surface 141 of bumper 140 may be coated with or formed from a conductive material and connected to the source of electrosurgical energy (not shown) to serve as the return electrode. Alternatively, proximal surface 143 may be formed from or coated with a conductive material and connected to the source of electrosurgical energy (not shown) to serve as the return electrode.

In use, with reference to FIGS. 5A and 5B, shaft 120 of electrosurgical instrument 100 is inserted into a body cavity, for example, an abdominopelvic cavity and positioned such that electrosurgical loop 130 and bumper 140 are positioned adjacent to tissue to be resected. A tissue grasping device 200 (e.g., tenaculum, forceps, etc.) including a handle assembly 210, a shaft assembly 220, and an end effector assembly 230 extending distally from shaft assembly 220, is also inserted into the body cavity for selectively grasping and manipulating tissue therewith. First and/or second actuators 113, 115 (FIG. 1) may also be actuated to define a desired dimension of electrosurgical loop 130.

Once electrosurgical instrument 100 is positioned as detailed above and, if desired, electrosurgical loop 130 extended or retracted to define a desired dimension, end effector 220 of tissue grasping device 200 may be utilized to grasp tissue and pull the tissue through electrosurgical loop 130 such that the tissue is pulled into contact with electrosurgical loop 130 and bumper 140.

As tissue grasping device 200 pulls tissue through electrosurgical loop 130, activation button 117 is actuated to energize electrosurgical loop 130. With electrosurgical loop 130 energized and tissue grasping device 200 pulling tissue through electrosurgical loop 130, electrosurgical loop 130 skives along an outer surface of tissue, guided by bumper 140, while a strip of tissue is resected from the tissue specimen and pulled through electrosurgical loop 130 via tissue grasping device 200. Resilient flexing of electrosurgical loop 130 and/or bumper 140 helps ensure electrosurgical loop 130 and bumper 140 are maintained in contact with tissue. Further, with bumper 140 sliding along the surface of the tissue specimen, electrosurgical loop 130 is inhibited from plunging through the tissue specimen and, instead, skives about the outer surface thereof to facilitate resection of the strip of tissue from the tissue specimen, similar to unraveling a ball of yarn.

As noted above, electrosurgical loop 130 may be cinched about the strip of tissue to cut the strip of tissue. In this manner, multiple strips of tissue may be resected from the tissue specimen instead of a single strip. In either configuration, the tissue specimen may be completely resected into strip(s) or may be resected sufficiently so as to enable minimally-invasive removal thereof.

The removal of a strip of tissue (e.g., as opposed to small tissue chips or chunks) is advantageous in that, e.g., operating time is reduced as well as the chance of cross-contamination with malignant or cancerous tissue. The strip of tissue can be removed, fed into a morcellator (not shown) for further break down, and/or placed into a specimen bag (not shown) for contained removal. Alternatively, the entire procedure detailed above may be performed within a specimen bag (not shown) for contained resection and removal.

Persons skilled in the art will understand that the structures and methods specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope or spirit of the disclosure. Additionally, the elements and features shown or described in connection with certain embodiments may be combined with the elements and features of certain other embodiments without departing from the scope of the present disclosure, and that such modifications and variations are also included within the scope of the present disclosure. Accordingly, the subject matter of the present disclosure is not limited by what has been particularly shown and described.

The invention may be described by reference to the following numbered paragraphs:-
1. An electrosurgical instrument, comprising:
   a housing;
   a shaft extending distally from the housing;
   an electrosurgical loop configured to connect to a source of energy and operably supported at a distal end portion of the shaft; and
   a bumper extending from the distal end portion of the shaft at an angle relative to the electrosurgical loop.
2. The electrosurgical instrument according to paragraph 1, wherein the electrosurgical loop defines an active electrode and the bumper defines a return electrode in a bipolar configuration.
3. The electrosurgical instrument according to paragraph 1, wherein the bumper is formed from a metal selected from the group consisting of copper, copper alloy, stainless steel, tungsten, platinum, niobium, titanium, steel, and aluminum.
4. The electrosurgical instrument according to paragraph 1, wherein the shaft defines a first axis and the bumper defines a second axis that is oriented at an angle from about 75 degrees to about 120 degrees relative to the first axis.
5. The electrosurgical instrument according to paragraph 1, wherein the bumper is pivotable relative to the shaft between a rest position and a pivoted position.
6. The electrosurgical instrument according to paragraph 5, further comprising a biasing element including a first end and a second end, the first end of the biasing element attached to the bumper, the second end of the biasing element attached to the shaft, wherein the bumper is configured to move to the pivoted position when in contact with a surface, and wherein the bumper is configured to automatically return to the rest position when not in contact with a surface.
7. The electrosurgical instrument according to paragraph 1, wherein the electrosurgical loop is movable between a contracted position, wherein the electrosurgical loop defines a first dimension, and an expanded position, wherein the electrosurgical loop defines a second, larger dimension.
8. The electrosurgical instrument according to paragraph 7, further comprising:
   an actuator operably coupled to the housing; and
   a drive assembly extending through the shaft and operably coupling the actuator with the electrosurgical loop,
   wherein actuation of the actuator moves the electrosurgical loop between the contracted and expanded positions.
9. The electrosurgical instrument according to paragraph 1, wherein the housing includes an activation button on a surface thereof, the activation button configured to selectively energize the electrosurgical loop with the source of electrical energy.
10. The electrosurgical instrument according to paragraph 1, wherein the bumper defines a blunt free end.
11. The electrosurgical instrument according to paragraph 1, wherein a distal tissue contacting surface of the bumper defines one of a flat, smooth, rough, and textured surface.
12. A method of performing a surgical procedure, comprising:
   inserting an electrosurgical instrument including an electrosurgical loop and a bumper disposed at an angle relative to the electrosurgical loop into a body cavity;
   activating an activation button to energize the electrosurgical loop;
   drawing tissue from a tissue specimen through the electrosurgical loop to resect a strip of tissue from the tissue specimen; and
   sliding the bumper along a surface of the tissue specimen while drawing the tissue to facilitate resection of the strip of tissue.
13. The method according to paragraph 12, wherein drawing tissue includes:
   grasping tissue with a grasping device; and
   pulling tissue with the grasping device through the electrosurgical loop.
14. The method according to paragraph 12, further comprising actuating an actuator of the electrosurgical instrument to expand or contract the electrosurgical loop.
15. A surgical system, comprising:
   an electrosurgical instrument including:
      a housing;
      a shaft extending distally from the housing;
      an electrosurgical loop adapted to connect to a source of energy and operably supported at a distal end portion of the shaft; and
      a bumper extending from the distal end portion of the shaft at an angle relative to the electrosurgical loop; and
   a grasping device including a handle assembly, a shaft assembly, and an end effector assembly extending from the shaft assembly and configured for grasping tissue, wherein the grasping device is configured to pull tissue through the electrosurgical loop such that the tissue is pulled into contact with the electrosurgical loop.
16. The surgical system according to paragraph 15, wherein the electrosurgical loop defines an active electrode and the bumper defines a return electrode in a bipolar configuration.
17. The surgical system according to paragraph 15, wherein the bumper is formed from a metal selected from the group consisting of copper, copper alloy, stainless steel, tungsten, platinum, niobium, titanium, steel, and aluminum.
18. The surgical system according to paragraph 15, wherein the shaft defines a first axis and the bumper defines a second axis that is oriented at an angle from about 75 degrees to about 120 degrees relative to the first axis.
19. The surgical system according to paragraph 18, wherein the bumper is pivotable relative to the shaft between a rest position and a pivoted position.
20. The surgical system according to paragraph 15, wherein the electrosurgical loop is movable between a contracted position, wherein the electrosurgical loop defines a first dimension, and an expanded position, wherein the electrosurgical loop defines a second, larger dimension.

## Claims

1. An electrosurgical instrument, comprising:
a housing;
a shaft extending distally from the housing;
an electrosurgical loop configured to connect to a source of energy and operably supported at a distal end portion of the shaft; and
a bumper extending from the distal end portion of the shaft at an angle relative to the electrosurgical loop.

2. The electrosurgical instrument according to claim 1, wherein the electrosurgical loop defines an active electrode and the bumper defines a return electrode in a bipolar configuration.

3. The electrosurgical instrument according to claim 1, or 2 wherein the bumper is formed from a metal selected from the group consisting of copper, copper alloy, stainless steel, tungsten, platinum, niobium, titanium, steel, and aluminum.

4. The electrosurgical instrument according to claim 1, 2 or 3 wherein the shaft defines a first axis and the bumper defines a second axis that is oriented at an angle from about 75 degrees to about 120 degrees relative to the first axis.

5. The electrosurgical instrument according to any preceding claim, wherein the bumper is pivotable relative to the shaft between a rest position and a pivoted position.

6. The electrosurgical instrument according to claim 5, further comprising a biasing element including a first end and a second end, the first end of the biasing element attached to the bumper, the second end of the biasing element attached to the shaft, wherein the bumper is configured to move to the pivoted position when in contact with a surface, and wherein the bumper is configured to automatically return to the rest position when not in contact with a surface.

7. The electrosurgical instrument according to any preceding claim, wherein the electrosurgical loop is movable between a contracted position, wherein the electrosurgical loop defines a first dimension, and an expanded position, wherein the electrosurgical loop defines a second, larger dimension.

8. The electrosurgical instrument according to claim 7, further comprising:
an actuator operably coupled to the housing; and
a drive assembly extending through the shaft and operably coupling the actuator with the electrosurgical loop,
wherein actuation of the actuator moves the electrosurgical loop between the contracted and expanded positions.

9. The electrosurgical instrument according to any preceding claim, wherein the housing includes an activation button on a surface thereof, the activation button configured to selectively energize the electrosurgical loop with the source of electrical energy.

10. The electrosurgical instrument according to any preceding claim, wherein the bumper defines a blunt free end.

11. The electrosurgical instrument according to any preceding claim, wherein a distal tissue contacting surface of the bumper defines one of a flat, smooth, rough, and textured surface.

12. A surgical system, comprising:
an electrosurgical instrument as claimed in any preceding claim and
a grasping device including a handle assembly, a shaft assembly, and an end effector assembly extending from the shaft assembly and configured for grasping tissue, wherein the grasping device is configured to pull tissue through the electrosurgical loop such that the tissue is pulled into contact with the electrosurgical loop.
